# EUROPEAN PATENT APPLICATION

(11) **EP 3 415 634 A1**
(43) Date of publication of application: **19.12.2018**
(21) Application number: 18177499.3
(22) Date of filing: 13.06.2018
(51) Int. Cl.: C12Q 1/00

(54) **BIOSENSOR AND MEASURING METHOD USING THE SAME**

(30) Priority: 13.06.2017 JP 2017116048
(71) Applicant: ARKRAY, Inc., Kyoto-shi, Kyoto 601-8045 (JP)
(72) Inventor: FUKUDA, Kazuo, Kyoto, 602-0008 (JP)
(74) Representative: Dehns

(57) **Abstract**

Provided is a biosensor for measuring a target substance in a sample in the presence of an interfering substance,
wherein the biosensor comprises an electrode pair comprising a working electrode and a counter electrode, and reagents provided on at least the working electrode, wherein the reagents comprise a first oxidoreductase capable of oxidizing or reducing the target substance, at least one mediator capable of transferring electrons generated from an oxidation reduction reaction of the first oxidoreductase to the working electrode, and a second oxidoreductase capable of oxidizing or reducing the interfering substance, and
wherein the oxidation-reduction potential of the mediator is higher than the oxidation-reduction potential of the first oxidoreductase and lower than the oxidation-reduction potential of the second oxidoreductase.

## Description

### TECHNICAL FIELD

The present disclosure relates to a biosensor and a measuring method using the biosensor.

### BACKGROUND ART

Glucose sensors for quantifying glucose in a biological sample by use of an enzyme electrode comprising glucose oxidoreductase and a mediator in a reagent layer have been widely used. Conventional glucose sensors, however, when used for measuring a sample obtained from a subject treated with an infusion or dosing containing an interfering substance such as ascorbic acid, have the problem of exhibiting a glucose measurement value which is lower or higher than an actual blood-sugar level. The reason for this is because the glucose oxidoreductase reacts with not only glucose, but also the interfering substance such as ascorbic acid and furthermore also directly reacts with the mediator, and the measured current value is a value affected by the reaction with the interfering substance such as ascorbic acid, thereby making it difficult to accurately measure the glucose concentration. Such a problem has been caused in not only glucose sensors, but also other biosensors such as lactic acid sensors for measuring lactic acid.

A measuring method has been then proposed in which the influence of an interfering substance such as ascorbic acid is reduced. Patent Document 1 discloses a measuring method including subtracting, from the charge obtained by a first electrode pair, the charge obtained by use of a second electrode coated with only a mediator, to thereby substantially remove the error due to the oxidation state of a redox mediator or remove the background current due to a diffusible redox mediator. The method described in Patent Document 1, however, requires preparation of another electrode for measuring an oxidation-reduction substance with only the mediator, and has the problem of a need for production of an electrode by distinctively coating a narrow reaction area with a reagent and the problem of the inability of accurate measurement due to the occurrence of contamination, thereby resulting in difficulty of quality control.

Patent Document 2 discloses an electrochemical sensor in which at least one electrode pair includes an enzyme and an oxidation-reduction mediating agent (mediator) and at least another electrode does not include any enzyme and includes an oxidation-reduction mediating agent. This electrochemical sensor also requires preparation of another electrode for measuring an oxidation-reduction substance with only the oxidation-reduction mediating agent and has the same problems as those of the method described in Patent Document 1.

On the other hand, Patent Document 3 discloses a technique in which an electrode comprising glucose oxidase and ascorbic acid oxidase is used in a reagent layer, to oxidize ascorbic acid in a sample by ascorbic acid oxidase, resulting in a reduction in the influence of ascorbic acid on glucose concentration measurement. This technique, however, is a technique in which dissolved oxygen in the sample liquid is reduced according to oxidation of glucose in the sample liquid, to generate hydrogen peroxide, and such hydrogen peroxide is oxidized by the electrode to sense the oxidation current, and the technique is not a measurement technique by a reversible reaction utilizing a mediator. In addition, hydrogen peroxide is used as a mediator, and thus the electrode material is limited to platinum or the like.

Patent Document 4 also discloses a biosensor for measuring glucose and the like by a treatment of ascorbic acid in a sample with ascorbic acid oxidase. This technique, however, is one in which ascorbic acid is treated with ascorbic acid oxidase in a first reaction layer and thereafter an oxidation reduction reaction of glucose and the like is performed in a second reaction layer separated by a barrier membrane, and the technique is not practical because potassium ferricyanide is used as a mediator (mediator) and the barrier membrane is essential so as to allow the mediator not to react with ascorbic acid oxidase, thereby causing the electrode configuration to be complicated.

### REFERENCES

Patent Document 1: JP 4885508 B
Patent Document 2: WO 1998/35225
Patent Document 3: JP 11-023515 A
Patent Document 4: JP 3102613 B

### SUMMARY OF THE INVENTION

An aspect of the present invention is to provide a biosensor which has a simplified structure, which allows the influence of an interfering substance such as ascorbic acid in a sample to be reduced, and which can simply perform concentration measurement of a target substance such as glucose at a high sensitivity, as well as a measuring method using the biosensor.

The inventor has found that the current value accurately depending on the concentration of only a target substance such as glucose can be detected by a biosensor, in which reagents comprising an oxidoreductase (first oxidoreductase) which oxidizes a target substance such as glucose, an oxidoreductase (second oxidoreductase) which oxidizes an interfering substance such as ascorbic acid, and a mediator are provided on at least a working electrode. The oxidation-reduction potential of the mediator is higher than that of the first oxidoreductase and lower than that that of oxidation-reduction potential of the second oxidoreductase, which allows the interfering substance such as ascorbic acid to be treated with the second oxidoreductase but the electrons generated from this reaction is not transferred to the electrode by the mediator, whereas the electrons based on the oxidation reaction of the target substance such as glucose is transferred the electrode by the mediator.

One aspect of the present invention is to provide a biosensor for measuring a target substance in a sample in the presence of an interfering substance, the biosensor comprising an electrode pair comprising a working electrode and a counter electrode, and reagents provided on at least the working electrode, wherein
the reagents comprise a first oxidoreductase capable of oxidizing or reducing the target substance, at least one mediator capable of transferring electrons generated from an oxidation reduction reaction of the first oxidoreductase to the working electrode, and a second oxidoreductase capable of oxidizing or reducing the interfering substance, and
wherein the oxidation-reduction potential of the mediator is higher than the oxidation-reduction potential of the first oxidoreductase and lower than the oxidation-reduction potential of the second oxidoreductase.

Another aspect of the present invention is to provide the biosensor as described above, wherein the second oxidoreductase is capable of oxidizing or reducing the interfering substance in preference to the first oxidoreductase.

Another aspect of the present invention is to provide the biosensor as described above, wherein the target substance is glucose.

Another aspect of the present invention is to provide the biosensor as described above, wherein the first oxidoreductase is glucose oxidoreductase.

Another aspect of the present invention is to provide the biosensor as described above, wherein the glucose oxidoreductase is glucose dehydrogenase.

Another aspect of the present invention is to provide the biosensor as described above, wherein the glucose oxidoreductase is glucose oxidase.

Another aspect of the present invention is to provide the biosensor as described above, wherein the interfering substance is ascorbic acid.

Another aspect of the present invention is to provide the biosensor as described above, wherein the second oxidoreductase is ascorbic acid oxidoreductase.

Another aspect of the present invention is to provide the biosensor as described above, wherein the second oxidoreductase is ascorbic acid oxidase.

Another aspect of the present invention is to provide the biosensor as described above, wherein the mediator is a ruthenium complex.

Another aspect of the present invention is to provide the biosensor as described above, wherein the reagents are provided on at least the working electrode in the form of an intimate mixture.

Another aspect of the present invention is to provide the biosensor as described above, wherein the reagents are provided on both the working electrode and the counter electrode

Another aspect of the present invention is to provide the biosensor as described above, wherein the sample is blood.

Another aspect of the present invention is to provide a method of measuring the concentration of a target substance in a sample in the presence of an interfering substance, comprising:
allowing a biosensor in the sample to react with a sample comprising the target substance;
applying a potential, to measure a response current; and
calculating the concentration of the target substance, based on the response current,
wherein the biosensor comprises an electrode pair comprising a working electrode and a counter electrode, and reagents provided on at least the working electrode, wherein the reagents comprise a first oxidoreductase capable of oxidizing or reducing the target substance, at least one mediator capable of transferring electrons generated from an oxidation reduction reaction of the first oxidoreductase to the working electrode, and a second oxidoreductase capable of oxidizing or reducing the interfering substance, and
wherein the oxidation-reduction potential of the mediator is higher than the oxidation-reduction potential of the first oxidoreductase and lower than the oxidation-reduction potential of the second oxidoreductase.

Another aspect of the present invention is to provide the method as described above, wherein the target substance is glucose and/or the sample is blood.

According to the present invention, it may not be necessary to separately provide an electrode for measuring an interfering substance such as ascorbic acid and therefore the structure of a biosensor can be simplified, and it may not be necessary to coat each electrode with a different reagent and therefore the production cost can be reduced. In addition, the influence of contamination or the like can be eliminated and therefore the frequency of detection of an abnormal value or the like can be reduced. Furthermore, it may not be necessary to correct the measured value in consideration of the value of the interfering substance such as ascorbic acid and therefore measurement can be simply performed and also the problem of deterioration in performance due to excessive correction or the like may not be caused.

### BRIEF DESCRIPTION OF THE DRAWINGS

FIG. 1 is a process chart illustrating one example of a method for producing a biosensor according to one embodiment of the present invention, and (A) to (E) illustrate schematic diagrams of the biosensor in respective steps.
FIG. 2 is a schematic diagram illustrating one embodiment of a measuring apparatus provided with one embodiment of the biosensor of the present invention.
FIG. 3 is a flowchart illustrating one embodiment of a measurement program using the measuring apparatus provided with one embodiment of the biosensor of the present invention.
FIG. 4 is a graph representing the change in current value (deviation from the current value in the case of 45 mg/dL of glucose and no ascorbic acid), when a biosensor comprising an electrode comprising glucose oxidase and ascorbic acid oxidase in a reagent layer is used and the ascorbic acid (AsA) concentration in a sample comprising 45 mg/dL of glucose is changed. FIG. 4 also represents the results of evaluation using a biosensor comprising an electrode comprising no ascorbic acid oxidase in a reagent layer, as a control.
FIG. 5 is a graph representing the change in current value (deviation from the current value in the case of 45 mg/dL of glucose and no ascorbic acid), when a biosensor comprising an electrode comprising glucose dehydrogenase and ascorbic acid oxidase in a reagent layer is used and the ascorbic acid (AsA) concentration in a sample comprising 45 mg/dL of glucose is changed. FIG. 5 also represents the results of evaluation using a biosensor comprising an electrode comprising no ascorbic acid oxidase in a reagent layer, as a control.

### EMBODIMENTS FOR CARRYING OUT THE INVENTION

One embodiment of the biosensor of the present invention is a biosensor for measuring a target substance in a sample in the presence of an interfering substance, comprising an electrode pair comprising a working electrode and a counter electrode, and reagents provided on at least the working electrode, wherein the reagents comprise a first oxidoreductase capable of oxidizing or reducing the target substance, at least one mediator capable of transferring electrons generated from an oxidation reduction reaction of the first oxidoreductase to the working electrode, and a second oxidoreductase capable of oxidizing or reducing the interfering substance which is different from the target substance, and the oxidation-reduction potential of the mediator is higher than the oxidation-reduction potential of the first oxidoreductase and lower than the oxidation-reduction potential of the second oxidoreductase.

Hereinafter, the explanation will be made to embodiments.

### (Biosensor)

One embodiment of the biosensor of the present invention has an electrode pair comprising a working electrode (enzyme electrode) and a counter electrode. The biosensor may have an electrode pair of a three-electrode system comprising a reference electrode in addition to the working electrode and the counter electrode.

### (Electrode pair)

The electrodes such as the working electrode and the counter electrode are not particularly limited in terms of materials thereof as long as such materials have conductivity, and the electrodes are each formed using, for example, a metallic material such as gold (Au), platinum (Pt), silver (Ag), ruthenium (Ru) or palladium (Pd), or a carbon material typified by carbon such as graphite, carbon nanotube, graphene or mesoporous carbon. The counter electrode and the reference electrode may also be a silver/silver chloride electrode.

The electrodes can be each formed on, for example, an insulating base plate. The insulating base plate is formed by a thermoplastic resin such as polyetherimide (PEI), polyethylene terephthalate (PET) or polyethylene (PE), various resins (plastics) such as a polyimide resin or an epoxy resin, or an insulating material such as glass, ceramic or paper. The sizes and the thicknesses of the electrodes and the insulating base plate can be appropriately selected.

### (Reagents)

The reagents comprise a first oxidoreductase, a mediator and a second oxidoreductase. The reagents may also comprise a binder, a buffer solution, and the like described below.

The reagent is provided on at least the working electrode, or may be provided on both of the working electrode and the counter electrode. The reagents may be provided on not only the working electrode or the working electrode and the counter electrode, but also the base plate on the periphery of the working electrode or the working electrode and the counter electrode.

The reagents may be provided on the electrode(s) with the first oxidoreductase, the mediator and the second oxidoreductase being mixed, which allows the first oxidoreductase and the second oxidoreductase to uniformly react with the target substance and the interfering substance respectively, thereby enabling accurate measurement to be conducted. The phrase "provided in the form of an intimate mixture" means that the first oxidoreductase, the mediator and the second oxidoreductase are mixed in the state where the reagents are provided, and the first oxidoreductase, the mediator and the second oxidoreductase may be dropped separately on the electrode(s) and then mixed, or may be dropped on the electrode(s) in the form of a liquid with these being mixed in advance.

### (Sample)

The sample is not particularly limited as long as it is a sample comprising the target substance and may be a biological sample and examples thereof include blood and urine.

### (Target substance)

Examples of the target substance include glucose, sorbitol, fructose, cholesterol, ethanol, lactic acid and uric acid.

### (Interfering substance)

The interfering substance is a substance other than the target substance and is a substance which has an influence on the reaction of the target substance and the first oxidoreductase, and examples thereof include ascorbic acid and uric acid (when the target substance is not uric acid).

### (First oxidoreductase)

The first oxidoreductase may be any enzyme whose substrate is the target substance and which can oxidize/reduce the target substance, and examples thereof include an oxidoreductase containing at least one of pyrroloquinoline quinone (PQQ) and flavin adenine dinucleotide (FAD), as a catalytic subunit or a catalytic domain. When the target substance is glucose, examples of an oxidoreductase containing PQQ include PQQ glucose dehydrogenase (PQQGDH) and examples of an oxidoreductase containing FAD include cytochrome glucose dehydrogenase (CyGDH) or glucose oxidase (GOD) having an α subunit containing FAD. Other examples include glucose-3-dehydrogenase (Glucose-3-Dehydrogenase) derived from *Agrobacterium tumefasience.*

The first oxidoreductase may contain an electron transfer subunit or an electron transfer domain. When the target substance is glucose, examples of the electron transfer subunit include a subunit containing heme which has a function of giving and receiving electrons. Examples of the oxidoreductase comprising such a subunit containing heme include those containing cytochrome, and, for example, a fusion protein of glucose dehydrogenase or PQQGDH with cytochrome may be used. The fusion protein of PQQGDH with cytochrome, as an example of the subunit containing cytochrome, and the cytochrome domain of PQQGDH, as an example of the domain containing cytochrome, are disclosed in, for example, WO 2005/030807.

When the target substance is other than glucose, examples of the enzyme containing the electron transfer domain include cholesterol oxidase and quinoheme ethanol dehydrogenase (QHEDH (PQQ Ethanol dh)). Furthermore, as the electron transfer domain, a domain containing cytochrome having heme which has a function of giving and receiving electrons may be used. Examples include sorbitol dehydrogenase (Sorbitol DH), D-fructose dehydrogenase (Fructose DH), cellobiose dehydrogenase, lactic acid dehydrogenase and uric acid oxidase.

Accordingly, the biosensor of the present invention may be used as not only a glucose sensor, but also a cholesterol sensor, an ethanol sensor, a sorbitol sensor, a fructose sensor, a cellobiose sensor, a lactic acid sensor, a uric acid sensor, and the like.

### (Second oxidoreductase)

The second oxidoreductase may be any enzyme capable of oxidizing/reducing the interfering substance other than the target substance contained in the sample, in preference to the first oxidoreductase, and examples thereof include ascorbic acid oxidase when the interfering substance is ascorbic acid. When the interfering substance is uric acid, other examples include uric acid oxidase, but are not limited thereto.

### (Mediator)

The mediator included in the reagents is a mediator which receives electrons generated by a reaction of the target substance and the first oxidoreductase and which also transfers such electrons to an electrode, and which has an oxidation-reduction potential higher than the oxidation-reduction potential of the first oxidoreductase and lower than the oxidation-reduction potential of the second oxidoreductase.

The oxidation-reduction potential is a value representing the intensity of the oxidation power or the reduction power of a substance and is an index for determination of whether the solution of the substance tends to be oxidized or reduced. As described below, the oxidoreductase and the mediator have respective specific oxidation-reduction potentials.

In an electrochemical biosensor, the electron transfer direction is determined based on the oxidation-reduction potentials of the electrodes, the mediator, and the reagent, and electrons flow from a lower oxidation-reduction potential to a higher oxidation-reduction potential. Accordingly, such a mediator is used, thereby transferring electrons generated by a reaction of the first oxidoreductase to the mediator, but not transferring electrons generated by a reaction of the second oxidoreductase to the mediator, resulting in transferring of only electrons received from the first oxidoreductase to the mediator.

The reagents contain the second oxidoreductase, and therefore electrons from the interfering substance can be transferred with the second oxidoreductase in preference to the mediator, thereby preventing direct transferring of electrons from the interfering substance to the mediator. Accordingly, the electrode can receive only electrons generated by a reaction of the first oxidoreductase, and therefore the current value (not affected by the interfering substance) reflecting the concentration of the target substance contained in the sample can be detected.

Such a mediator may be appropriately selected depending on the types of the first oxidoreductase and the second oxidoreductase. For example, when the first oxidoreductase is an oxidoreductase which uses FAD as a co-enzyme, like a glucose oxidase (GOD) and glucose dehydrogenase (GDH), the oxidation-reduction potential is -219 mV, and when the second oxidoreductase includes a copper ion at the active center, like ascorbic acid oxidase (AsOD), the oxidation-reduction potential is 340 mV. Therefore, a mediator having an oxidation-reduction potential between the above values is selected.

For example, in Examples described below, the oxidation-reduction potential is in the order of GOD, GDH < Ru < 1-Methoxy-PES < AsOD (that of [Ru (NH₃)₆]Cl₃ is about 100 mV and that of 1-Methoxy-PMS is 63 mV), and therefore no electrons flow from AsOD to the mediator whereas electrons flow from glucose oxidoreductase (GOD, GDH) reactive with glucose, to the mediator (Ru or 1-Methoxy-PES).

Thus, as the mediator, one having an oxidation-reduction potential higher than the oxidation-reduction potential of the first oxidoreductase and lower than the oxidation-reduction potential of the second oxidoreductase is selected. The mediator may be any non-catalytic compound which receives electrons from the first oxidoreductase and thus is reduced and re-oxidized in the electrode, and examples thereof include ruthenium compounds, quinone compounds (for example, 1,4-Naphthoquinone, VK3, 9,10-Phenanthrenequinone, 1,2-Naphthoquinone, p-Xyloquinone, Methylbenzoquinone, 2,6-Dimethylbenzoquinone, Sodium 1,2-Naphthoquinone-4-sulfonate, 1,4-Anthraquinone, Tetramethylbenzoquinone and Thymoquinone), phenylenediamine compounds (for example, N,N-Dimethyl-1,4-phenylenediamine and N,N,N',N'-tetramethyl-1, 4-phenylenediamine dihydrochloride), 1-Methoxy-PMS (1-Methoxy-5-methylphenazinium methylsulfate), Coenzyme Q0, AZURE A Chloride, Phenosafranin, 6-Aminoquinoxaline, and Tetrathiafulvalene, and these may be used singly or in combination of two or more thereof.

In particular, a complex comprising an oxidized type metal atom and a ligand may be used, and for example, a ruthenium complex comprising trivalent ruthenium (Ru (III)) and a ligand may be used.

The contents of the first oxidoreductase and the second oxidoreductase in the reagent layer (sensing layer) of the biosensor of the present invention may be appropriately determined depending on the types of the target substance and an interfering substance, the amount of the first oxidoreductase is, for example, from 1 to 10 U, from 1 to 5 U, or from 1 to 3U because the first oxidoreductase may be contained sufficiently for the target substance, and the amount of the second oxidoreductase is, for example, from 0.5 to 10 U, from 0.5 to 5 U, or from 0.5 to 2.5 U because the second oxidoreductase may be contained sufficiently relative to the amount of the interfering substance.

The content of the mediator in the reagent layer (sensing layer) may be any content higher than those of the first and second oxidoreductases, can be appropriately determined depending on the type and the like of a measurement sample, and is, for example, from 10 mmol to 100 mmol, from 10 mmol to 50 mmol, or from 15 mmol to 20 mmol per square centimeter of the surface area of the reagent layer.

### (Other component(s))

The reagent layer includes the first and second oxidoreductases and the mediator and may contain a binder in addition thereto. For example, in an electrode production process, a reagent layer preparation liquid can be prepared as an ink for screen printing, the ink containing the first and second oxidoreductases, the mediator, a binder and a solvent or a dispersion medium, and pattern printing on a base material such as a resin by screen printing can be made, to form an electrode, thereby producing electrode efficiently with a decrease in the variation between the sensors.

As the binder which may be used in the reagent layer, for example, a resin binder such as a butyral resin-based or a polyester resin-based binder may be used, or a layered inorganic compound disclosed in WO2005/043146 may also be used. Examples of the layered inorganic compound include one in which polyhedrons of inorganics are planarly continued to form a sheet structure and the sheet structure is further stacked in a layered manner to form a crystalline structure. Examples of the polyhedron form include a tetrahedron sheet and an octahedron sheet, and specific examples include a Si tetrahedron and an Al octahedron. Examples of such a layered inorganic compound include layered clay mineral, hydrotalcite, bentonite, smectite, vermiculite, halloysite, kaolin mineral, and mica. As such a layered inorganic compound, for example, a commercially available product may be used, and any of Rusentite SWN and Rusentite SWF (synthetic hectorite), and ME (fluoromica) produced by Co-op Chemical Co., Ltd., Sumecton SA (synthetic saponite) produced by KUNIMINE INDUSTRIES CO., LTD., Thixopy W (synthetic hectorite) and KYOWAAD 500 (synthetic hydrotalcite) produced by Kyowa Chemical Co., Ltd., LAPONITE (synthetic hectorite) produced by Laporte Industries Ltd., natural bentonite produced by NACALAI TESQUE, INC., or MULTIGEL (bentonite) produced by HOJUN.,Co., Ltd. may be used. The content of the binder in the reagent layer is, for example, in the range from 0.003 to 30 mg per square centimeter of the area of the reagent layer.

The reagent layer may also additionally include any additive such as a buffer and a surfactant, and the content of such any additive may be appropriately set.

The buffer may be an amine-based buffer, and examples thereof include Tris, ACES, CHES, CAPSO, TAPS, CAPS, Bis-Tris, TAPSO, TES, Tricine and ADA. These substances may be used singly or in combination of two or more thereof. A buffer having a carboxyl group may also be used as the buffer, and examples thereof include an acetic acid-Na acetate buffer, a malic acid-Na acetate buffer, a malonic acid-Na acetate buffer and a succinic acid-Na acetate buffer.

Examples of the surfactant include Triton X-100, sodium dodecyl sulfate, perfluorooctanesulfonic acid or sodium stearate, alkylaminocarboxylic acid (or a salt thereof), carboxybetaine, sulfobetaine and phosphobetaine.

### (Method for preparing biosensor)

One embodiment of production method of the biosensor of the present invention is as follows, for example. That is, a metal layer serving as each of the working electrode and the counter electrode is formed on one surface of the insulating base plate. For example, a film of a metallic material is formed on one surface of a film-shaped insulating base plate having a predetermined thickness (for example, about 100 µm) according to screen printing, physical vapor deposition (PVD, for example, sputtering), or chemical vapor deposition (CVD), thereby forming a metal layer having a desired thickness (for example, about 30 nm). An electrode layer formed by a carbon material may also be formed instead of the metal layer. Then, the electrode(s) may be coated with the reagent layer preparation liquid comprising the first and second oxidoreductases and the mediator, and dried, to thereby place the reagent layer on the electrode(s) (at least the working electrode, and in one embodiment, both the working electrode and the counter electrode).

When the working electrode of the biosensor is brought into contact with a sample containing the target substance, electrons generated by an oxidation reaction or a reduction reaction of the target substance by the first oxidoreductase are transferred to the mediator, and the mediator is reduced. An oxidation potential is then applied to the working electrode, thereby oxidizing a reduction type mediator on the working electrode surface, to thereby generate an oxidation current. The current value is not affected by any substance other than the target substance in the sample, and therefore the concentration of the target substance in the sample can be measured based on the current value.

That is, the mediator for use in the biosensor has an oxidation-reduction potential higher than the oxidation-reduction potential of the first oxidoreductase and lower than the oxidation-reduction potential of the second oxidoreductase, and therefore electrons generated by a reaction of the first oxidoreductase are transferred to the mediator, but electrons generated by a reaction of the second oxidoreductase are not transferred to the mediator, and as a result, only electrons received from the first oxidoreductase are transferred to the mediator. Accordingly, the electrodes can receive only electrons generated by a reaction of the first oxidoreductase, and therefore a current value reflecting the concentration of the target substance contained in the sample (not affected by the interfering substance) can be detected and the concentration of the target substance in the sample can be accurately measured based on the current value.

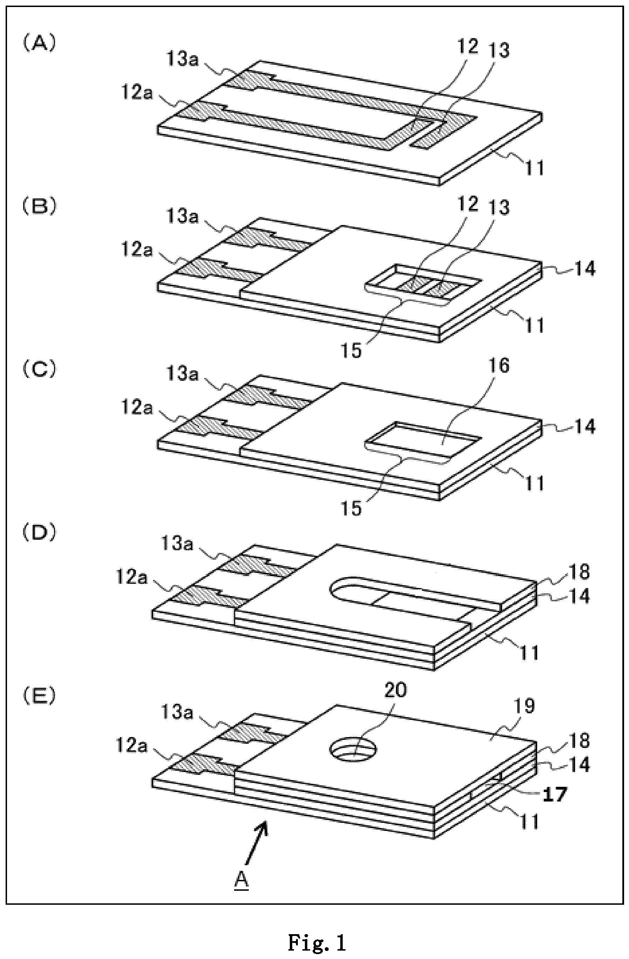

Hereinafter, one example of the biosensor will be described based on FIG. 1. FIG. 1(A) to FIG. 1(E) are perspective diagrams illustrating a series of steps of producing the biosensor. The biosensor of the present invention is not limited to the following embodiment.

As illustrated in FIG. 1(E), a biosensor A includes a base plate 11, an electrode pair composed of a working electrode 12 having a lead section 12a and a counter electrode 13 having a lead section 13a, an insulating layer 14, a reagent layer 16 comprising first and second oxidoreductases and a mediator, a spacer 18 having an opening, and a cover 19 having a through-hole 20. As illustrated in FIG. 1(B), a detection section 15 is provided on the base plate 11, and the working electrode 12 and the counter electrode 13 are disposed on the detection section 15 in parallel with the width direction of the base plate 11. Each one end of the electrode pair serves as each of the lead sections 12a and 13a and is disposed so as to be perpendicular to each other end on the detection section 15 (FIG. 1(A)). A region between the working electrode 12 and the counter electrode 13 corresponds to an insulating section. As illustrated in FIG. 1(B), the insulating layer 14, excluding the lead sections 12a and 13a and the detection section 15, is laminated on the base plate 11 provided with such an electrode system, and the reagent layer 16 is provided on the detection section 15 on which the insulating layer 14 is not laminated. As illustrated in FIG. 1(D), a spacer 18 having an opening in a region corresponding to the detection section 15 is then disposed on the insulating layer 14. A cover 19 having a through-hole 20, which is provided on a location corresponding to a portion of the opening of the spacer, is disposed on the spacer 18 (FIG. 1(E)). In such a biosensor 1, a space portion of the opening, which is a space portion sandwiched among the reagent layer 16 and the insulating layer 14, and the cover 19, serves as a sample-feeding section 17 of a capillary structure. The through-hole 20 then serves as an air hole for sucking the sample by a capillary phenomenon.

Such a biosensor may be produced as follows, for example.

First, as illustrated in FIG. 1(A), an electrode system comprising the working electrode 12 having the lead section 12a and the counter electrode 13 having the lead section 13a is formed on the base plate 11.

Subsequently, as illustrated in FIG. 1(B), the insulating layer 14 is formed on the base plate 11 on which the electrode pairs 12 and 13 are formed. The insulating layer is formed on the base plate 11 excluding the lead sections 12a and 13a of the electrodes, and the detection section 15. The insulating layer 14 may be formed by, for example, subjecting an insulation paste with an insulating resin dissolved in a solvent, to printing on the base plate 11, and subjecting the resultant to a heating treatment or an ultraviolet treatment. Examples of the insulating resin include polyester, a butyral resin and a phenol resin, and examples of the solvent include carbitol acetate and a dibasic acid ester-based mixed solvent (DBE solvent).

Next, as illustrated in FIG. 1(C), the reagent layer 16 is formed on the base plate 11 and the electrodes 12 and 13 on the detection section 15 on which the insulating layer 14 is not formed. The reagent layer 16 may be formed by, for example, preparing a dispersion liquid in which the first and second oxidoreductases, the mediator, and if necessary a buffer and a binder are dispersed, dispensing the dispersion liquid to the detection section 15 and drying it. As the solvent for use in preparation of the dispersion liquid, for example, water, a buffer solution alcohol, N,N-dimethylformamide (DMF) or dimethylsulfoxide (DMSO) may be used.

Next, as illustrated in FIG. 1(D), the spacer 18 is disposed on the insulating layer 14. As illustrated in the drawing, a region of the spacer 18, corresponding to the reagent layer 16, serves as an opening. As the material of the spacer 18, for example, a resin film or tape can be used. A double-sided adhesive tape can allow for not only adhesion with the insulating film 14, but also easy adhesion with the cover 19 described below. Besides this, the spacer may also be formed by a procedure such as resist printing.

Next, as illustrated in FIG. 1(E), the cover 19 is disposed on the spacer 18. The material of the cover 19 is not particularly limited, any of various plastics may be used therefor, and examples thereof include transparent resins such as PET.

The method of using the biosensor 1 will be described with reference to an example where the sample is whole blood, the target substance is glucose, the first oxidoreductase is glucose dehydrogenase, the interfering substance contained in the sample is ascorbic acid, the second oxidoreductase is ascorbic acid oxidase, and the mediator is a ruthenium (III) compound.

First, the whole blood sample is brought into contact with one end of the opening 17 of the biosensor 1. The opening 17 has a capillary structure as described above and the cover 19 corresponding to other end of the opening 17 is provided with an air hole 20, and therefore the sample is sucked inside by a capillary phenomenon. The sample sucked reaches the surface of the reagent layer 16 provided on the detection section 15. Ascorbic acid in the sample, reaching the surface, then preferentially reacts with ascorbic acid oxidase in the reagent layer 16, and is converted into dehydroascorbic acid. On the other hand, glucose in the sample preferentially reacts with glucose dehydrogenase, and is converted into gluconolactone. The ruthenium (III) compound is reduced by electrons generated here, thereby producing a ruthenium (II) compound. A positive potential is applied to the electrodes, thereby performing giving and receiving of electrons between the ruthenium (II) compound present in the reagent layer 16 and the electrodes positioned under the reagent layer 16, to allow an oxidation current to flow. The glucose concentration can be measured based on the current. On the other hand, electrons are not transferred to the ruthenium (III) compound from dehydroascorbic acid generated by the reaction of ascorbic acid oxidase, according to the above oxidation-reduction potential relationship, and are not sensed in the electrodes, and therefore the oxidation current accurately reflects the concentration of glucose.

After a lapse of a certain time from feeding of the whole blood sample, the voltage is applied between the working electrode 12 and the counter electrode 13 by a tool for voltage application, and the oxidation current flowing is detected by a tool or the like for electric signal measurement via the lead section 12a of the working electrode 12. The oxidation current value is proportional to the concentration of glucose in the sample, and therefore can be used for calculation of the glucose concentration by the calculation tool, thereby allowing the glucose concentration in the sample to be determined.

The voltage to be applied to the working electrode 12 is a voltage, which is higher than the oxidation-reduction potential of the mediator and lower than the oxidation-reduction potential of the interfering substance, may be any positive voltage relative to the counter electrode, therefore may be appropriately set depending on the types of the mediator and the interfering substance, and is, for example, from 100 to 340 mV, 150 to 300 mV, or 150 to 250 mV when the mediator is a ruthenium compound and the second oxidoreductase is ascorbic acid oxidase in Examples described below. The voltage may be applied to the electrode system after holding with no application for a predetermined time after the contact with the sample, or the voltage may be applied to the electrode system at the same time as the contact with the sample. The time for holding with no application is, for example, 30 seconds or less or 10 seconds or less.

### (Apparatus)

Next, one embodiment of a measuring apparatus provided with the biosensor will be described with reference to the drawings. While one embodiment of a measuring apparatus provided with the glucose sensor is here exemplified, the measuring apparatus provided with the biosensor of the present invention is not limited to the following embodiment.

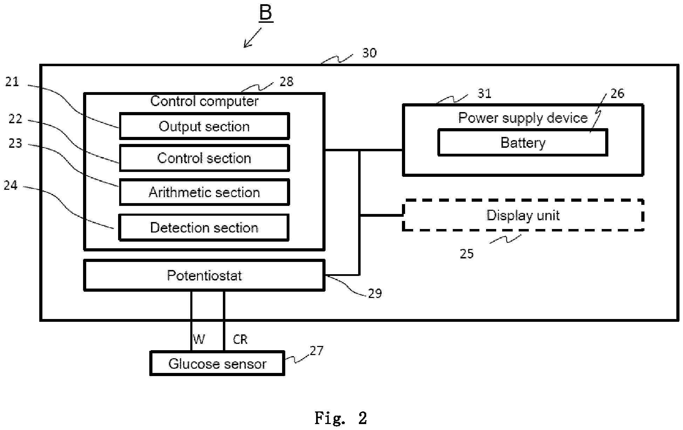

FIG. 2 illustrates an example of the configuration of main electronic components included in a measuring apparatus B. A control computer 28, a potentiostat 29 and a power supply device 31 are provided on a base plate 30 housed in a housing. The control computer 28 includes, as hardware, a processor such as CPU (central processing unit), memory (RAM (Random Access Memory), a recording medium such as ROM (Read Only Memory)), and a communication unit. When the processor loads a program stored in the recording medium (for example, the ROM) to the RAM, and executes the program, the control computer 28 functions as an apparatus comprising an output section 21, a control section 22, an arithmetic section 23 and a detection section 24. The control computer 28 may also include an auxiliary memory such as a semiconductor memory (EEPROM or flash memory) or a hard disk.

The control section 22 controls the timing for applying a voltage, the value of the voltage to be applied, and the like. The power supply device 31 has a battery 26, and supplies electricity to the control computer 28 and the potentiostat 29 for operation. It is also possible to dispose the power supply device 31 outside the housing. The potentiostat 29 is a device which maintains the potential of the working electrode at a constant value with respect to the potential of the reference electrode, and the potentiostat 29, which is controlled by the control section 22, applies a predetermined voltage (voltage higher than the oxidation-reduction potential of the mediator and lower than the oxidation-reduction potential of the interfering substance) between the counter electrode and the working electrode of the glucose sensor 27 by use of terminals CR and W, measures the response current of the working electrode, obtained at the terminal W, and sends the measurement results of the response current to the detection section 24.

The arithmetic section 23 calculates the concentration of the target substance, based on the current value detected, and stores it. The output section 21 carries out data communication between the output section 21 and a display unit 25 and sends the results of the concentration of the target substance provided by the arithmetic section 23, to the display unit 25. The display unit 25 is capable of displaying, for example, the calculated results of the glucose concentration which is received from the measuring apparatus B, on a display screen in a predetermined format.

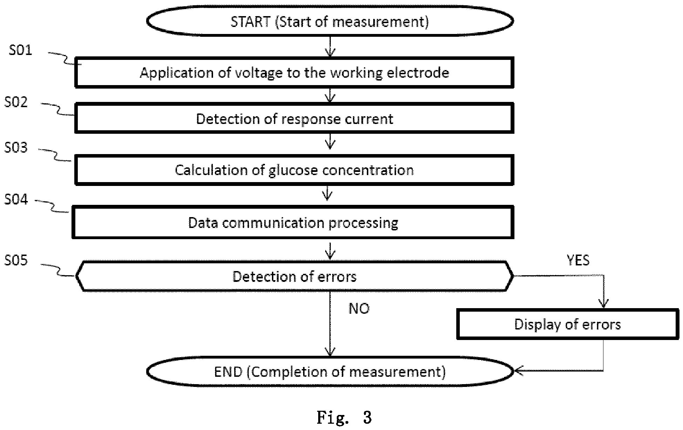

FIG. 3 is a flowchart illustrating an example of the processing sequence of the glucose concentration measurement carried out by the control computer 28. When the CPU (control section 22) of the control computer 28 receives an instruction to start the measurement of the glucose concentration, the control section 22 controls the potentiostat 29 to apply a predetermined voltage to the working electrode and starts measuring the response current from the working electrode (step S01). It is noted that sensing of installation of a sensor to the measuring apparatus may be used as the instruction to start the concentration measurement.

Next, the potentiostat 29 measures the response current obtained by voltage application, namely, a current based on transfer of electrons derived from the target substance (glucose) in the sample to the electrodes, for example, a steady-state current after one to 20 seconds from voltage application and sends such a current to the detection section 24 (step S02).

The arithmetic section 23 carries out arithmetic processing based on the current value and calculates the glucose concentration (step S03). For example, the calculation formula of the glucose concentration or the data of the calibration curve of the glucose concentration are preinstalled to the arithmetic section 23 in the control computer 28, and such calculation formula and calibration curve are used to calculate the glucose concentration.

The output section 21 sends the calculation results of the glucose concentration to the display unit 25 through a communication link provided between the output section 21 and the display unit 25 (step S04). Thereafter, the control section 22 determines whether or not there are any measurement errors detected (step S05), completes the measurement if there is no error, and displays the glucose concentration on the display section. If there are any errors, a notification of error is displayed, and then the flow sequence illustrated in FIG. 3 is completed. The calculation results can also be stored in the arithmetic section 23, and the calculation results can also be subsequently called, and displayed on the display section and confirmed. While detection of whether or not there are any measurement errors by the control section 22 (step S05) is performed after the calculation results are sent to the display unit 25 (step S04), the order of these steps may also be exchanged.

### EXAMPLES

Hereinafter, the present invention will be more specifically described with reference to Examples, but the present invention is not limited to the embodiments of the following Examples.

### (1) Production of biosensor

The biosensor was produced according to the following procedure, as illustrated in FIG. 1. First, as illustrated in FIG. 1(A), a base plate made of PET (length: 50 mm, width: 6 mm, thickness: 250 µm) was prepared as an insulating base plate 11 of a glucose sensor, and a carbon electrode pair comprising a working electrode 12 and a counter electrode 13 each having a lead section was formed on one surface thereof according to screen printing.

Next, as illustrated in FIG. 1(B), an insulating layer 14 was formed on the electrode pair. First, insulating resin polyester was dissolved in carbitol acetate as a solvent so as to be in a concentration of 75% (wt), thereby preparing an insulating paste, and the insulating paste was applied onto the electrodes according to screen printing. The printing conditions were as follows: a 300-mesh screen was used; the squeegee pressure was 40 kg and the amount of printing was 0.002 mL per square centimeter of the electrode area. Herein, a detection section 15, and lead sections 12a and 13a were not subjected to screen printing. A heating treatment at 155°C for 20 minutes was then performend to form the insulating layer 14.

Then, as illustrated in FIG. 1(C), a reagent layer 16 was formed on a detection section 15 on which the insulating layer 14 was not formed. Two reagent layers: one where glucose oxidase was used as the glucose oxidoreductase and other where glucose dehydrogenase was used as the glucose oxidoreductase; were prepared.

A preparation liquid for preparing a reagent layer for glucose oxidase was prepared as follows. First, a ruthenium compound liquid (pH: 7.5) comprising 0.3% (wt) of synthetic smectite (trade name "Rusentite SWN", produced by Co-op Chemical Co., Ltd.), 6.0% (wt) of a ruthenium compound ([Ru (NH₃)₆]Cl₃, produced by Dojindo Molecular Technologies, Inc.), sodium acetate and succinic acid was prepared. Glucose oxidase (2.0 Unit/dL) and ascorbic acid oxidase (1.0 Unit/dL) were added thereto, to provide a preparation liquid for preparing the reagent layer. Here, a preparation liquid for preparing the reagent layer, comprising no ascorbic acid oxidase, was also prepared as a control.

On the other hand, a preparation liquid for preparing a reagent layer for glucose dehydrogenase was prepared as follows. First, a ruthenium compound liquid (pH: 7.5) comprising 0.3% (wt) of synthetic smectite (trade name: "Rusentite SWN", produced by Co-op Chemical Co., Ltd.), 6.0% (wt) of a ruthenium compound ([Ru (NH₃)₆]Cl₃, produced by Dojindo Molecular Technologies, Inc.), 0.3% (wt) of 1-Methoxy-PES, sodium acetate and succinic acid was prepared. Glucose dehydrogenase (3.0 Unit/dL) and ascorbic acid oxidase (2.5 Unit/dL) were added thereto, to provide a preparation liquid for preparing the reagent layer. Here, a preparation liquid for preparing the reagent layer, comprising no ascorbic acid oxidase, was also prepared as a control.

Each of the preparation liquids for preparing the reagent layer was dispensed to a 1.0-µL detection section 15. The surface area of the detection section 15 was about 0.6 cm², and the surface area of each of the electrodes 12 and 13 in the detection section 15 was about 0.12 cm². The resultant was then dried at 30°C, thereby forming each reagent layer 16.

As illustrated in FIG. 1(D), a spacer 18 having an opening was disposed on the insulating layer 14. Then, as illustrated in FIG. 1(E), a cover 19 having a through-hole 20 serving as an air hole was disposed on the spacer 18, thereby producing a biosensor. The space of the opening in the spacer 18, sandwiched between the cover 19 and the insulating layer 14, had a capillary structure, and thus was adopted as a sample-feeding section 17.

### (2) Measurement of objective components

A whole blood sample comprising 45 mg/dL of glucose and ascorbic acid (AsA) in each concentration (0, 5, 10 or 15 mg/dL) was prepared.

Each whole blood sample was then fed through the sample-feeding section of the biosensor and subjected to a reaction for one second and thereafter application of a voltage of 200 mV for six seconds. The current value after six seconds from application of the voltage was sampled, the bias (%) of each concentration relative to the response current value in each ascorbic acid concentration and the response current value in an ascorbic acid concentration of 0 mg/dL was plotted, and the dependence of the ascorbic acid (AsA) concentration was analyzed.

### (3) Results

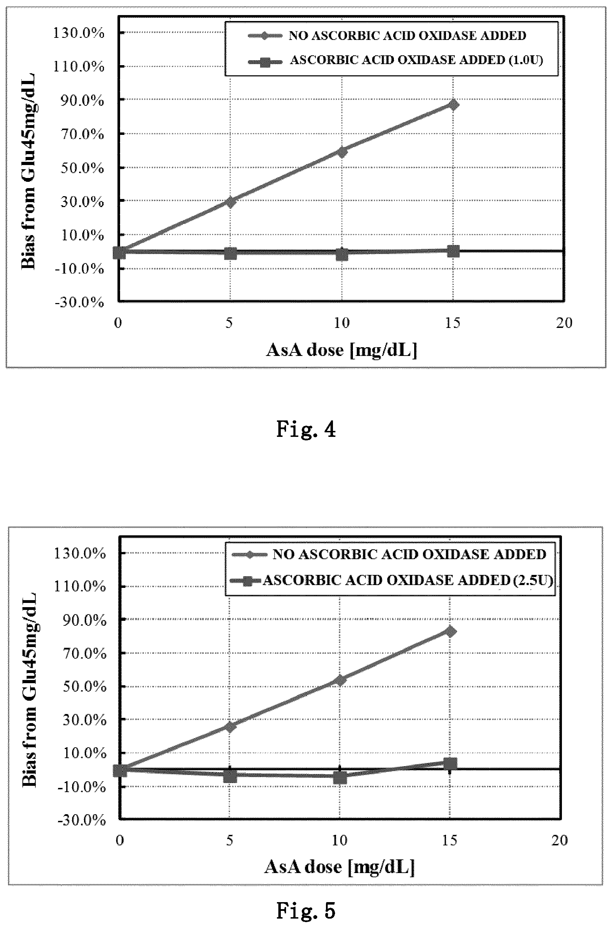

FIG. 4 represented the change in current value (the degree of deviation from the current value in the case of 45 mg/dL of glucose and no ascorbic acid), when a biosensor comprising an electrode comprising glucose oxidase and ascorbic acid oxidase in a reagent layer was used and the ascorbic acid (AsA) concentration in a glucose-containing sample was changed. As a result, the current value was not changed even if the ascorbic acid concentration was increased. On the other hand, when a biosensor comprising an electrode with a reagent layer comprising no ascorbic acid oxidase was used, the current value was increased as the ascorbic acid concentration was increased. The same results were obtained even in the case in FIG. 5, where an electrode comprising glucose dehydrogenase and ascorbic acid oxidase in a reagent layer was used.

It has been found from these results that a biosensor comprising an electrode comprising glucose oxidoreductase as a first oxidoreductase, ascorbic acid oxidase as a second oxidoreductase, and a mediator having an oxidation-reduction potential higher than the oxidation-reduction potential of the glucose oxidoreductase and lower than the oxidation-reduction potential of the ascorbic acid oxidase as the second oxidoreductase, in a reagent layer, enables glucose and the like to be quantified without being affected by an interfering substance such as ascorbic acid.

Accordingly, it has been found that a biosensor for measuring a target substance in a sample in the presence of an interfering substance, comprising an electrode pair comprising a working electrode and a counter electrode, and a reagent provided on at least the working electrode, wherein the reagent includes a first oxidoreductase capable of oxidizing or reducing the target substance, a second oxidoreductase capable of oxidizing or reducing the interfering substance, and at least one mediator capable of transferring electrons generated from an oxidation reduction reaction of the first oxidoreductase to the working electrode, and the oxidation-reduction potential of the mediator is higher than the oxidation-reduction potential of the first oxidoreductase and lower than the oxidation-reduction potential of the second oxidoreductase, can be used to quantify the target substance without being affected by the interfering substance.

### DESCRIPTION OF SYMBOLS

- A: biosensor
- 11: base plate
- 12: working electrode
- 12a: lead section
- 13: counter electrode
- 13a: lead section
- 14: insulating layer
- 15: detection section
- 16: reagent layer
- 17: opening
- 18: spacer
- 19: cover
- 20: air hole
- B: measuring apparatus
- 21: output section
- 22: control section
- 23: arithmetic section
- 24: detection section
- 25: display section unit
- 26: battery
- 27: glucose sensor
- 28: control computer
- 29: potentiostat
- 30: base plate
- 31: power supply device
- CR, W: terminal

## Claims

1. A biosensor for measuring a target substance in a sample in the presence of an interfering substance, the biosensor comprising an electrode pair comprising a working electrode and a counter electrode, and reagents provided on at least the working electrode, wherein
the reagents comprise a first oxidoreductase capable of oxidizing or reducing the target substance, at least one mediator capable of transferring electrons generated from an oxidation reduction reaction of the first oxidoreductase to the working electrode, and a second oxidoreductase capable of oxidizing or reducing the interfering substance, and
wherein the oxidation-reduction potential of the mediator is higher than the oxidation-reduction potential of the first oxidoreductase and lower than the oxidation-reduction potential of the second oxidoreductase.

2. The biosensor according to claim 1, wherein the second oxidoreductase is capable of oxidizing or reducing the interfering substance in preference to the first oxidoreductase.

3. The biosensor according to claim 1 or claim 2, wherein the target substance is glucose.

4. The biosensor according to claim 3, wherein the first oxidoreductase is glucose oxidoreductase.

5. The biosensor according to claim 4, wherein the glucose oxidoreductase is glucose dehydrogenase.

6. The biosensor according to claim 4, wherein the glucose oxidoreductase is glucose oxidase.

7. The biosensor according to any one of claims 1 to 6, wherein the interfering substance is ascorbic acid.

8. The biosensor according to claim 7, wherein the second oxidoreductase is ascorbic acid oxidoreductase.

9. The biosensor according to claim 8, wherein the second oxidoreductase is ascorbic acid oxidase.

10. The biosensor according to any one of claims 1 to 9, wherein the mediator is a ruthenium complex.

11. The biosensor according to any one of claims 1 to 9, wherein the reagents are provided on at least the working electrode in the form of an intimate mixture.

12. The biosensor according to any one of claims 1 to 11, wherein the reagents are provided on both the working electrode and the counter electrode.

13. The biosensor according to any one of claims 1 to 12, wherein the sample is blood.

14. A method of measuring the concentration of a target substance in a sample in the presence of an interfering substance, comprising:
allowing a biosensor to react with the sample comprising the target substance;
applying a potential to measure a response current; and
calculating the concentration of the target substance based on the response current,
wherein the biosensor comprises an electrode pair comprising a working electrode and a counter electrode, and reagents provided on at least the working electrode, wherein the reagents comprise a first oxidoreductase capable of oxidizes or reduces the target substance, at least one mediator capable of transferring electrons generated from an oxidation reduction reaction of the first oxidoreductase to the working electrode, and a second oxidoreductase capable of oxidizing or reducing the interfering substance, and
wherein the oxidation-reduction potential of the mediator is higher than the oxidation-reduction potential of the first oxidoreductase and lower than the oxidation-reduction potential of the second oxidoreductase.

15. The method according to claim 14, wherein the target substance is glucose and/or the sample is blood.
